# EUROPEAN PATENT APPLICATION

(11) **EP 4 559 319 A1**
(43) Date of publication of application: **28.05.2025**
(21) Application number: 23842875.9
(22) Date of filing: 11.07.2023
(51) Int. Cl.: A23C 9/13, A23L 2/38, A23L 2/52, A23L 5/00, A23L 33/135

(54) **ACIDIC MILK BEVERAGE CONTAINING LIVE LACTIC ACID BACTERIUM**

(30) Priority: 19.07.2022 JP 2022114476
(71) Applicant: Kabushiki Kaisha Yakult Honsha, Tokyo, 105-8660 (JP); Fuji Oil Holdings Inc., Izumisano-shi, Osaka 598-8540 (JP)
(72) Inventor: NAKANO, Masatoshi, Tokyo 105-8660 (JP); MIIDA, Satoshi, Tokyo 105-8660 (JP); ASAI, Yuji, Izumisano-shi, Osaka 598-8540 (JP); HATSUTORI, Mitsuo, Izumisano-shi, Osaka 598-8540 (JP)
(74) Representative: Wächtershäuser & Hartz Patentanwaltspartnerschaft mbB
(86) International application number: PCT/JP2023/025567
(87) International publication number: WO 2024/018950

(57) **Abstract**

An acidic milk beverage containing live lactic acid bacterium characterized by containing an ultrafiltration membrane-treated material and/or an ethanol-precipitated material of a pea polysaccharide is an acidic milk beverage containing live lactic acid bacterium which is soybean allergen-free and which has improved viability of a lactic acid bacterium.

## Description

### Technical Field

The present invention relates to an acidic milk beverage containing live lactic acid bacterium having improved viability of a lactic acid bacterium.

### Background Art

Lactic acid bacteria have various effects, such as improvement of the intestinal flora, improvement of stool consistencies, improvement of the intestinal tract functions, protection against infection, immunostimulation and prevention of a cancer, and thus are used for producing lactic acid bacterium preparations as pharmaceutical products and for foods and drinks such as fermented milk, lactic acid bacteria beverages and cheeses. For these applications, lactic acid bacteria are most often cultured using mammalian milk as a medium. Lactic acid bacteria, however, generally differ in auxotrophy depending on the kinds thereof and do not proliferate well in a medium composed of mammalian milk only, and it is considered that even a bacterial strain having relatively excellent proliferative activity has to be cultured for several days in a medium composed of mammalian milk only, in order to obtain a fermented material having a sufficient acidity for producing fermented milk, a lactic acid bacteria beverage or the like.

Such culture of a lactic acid bacterium over a long period is a cause for a decrease in the viable cell count and thus cannot necessarily be considered as a preferable method as culture for producing a lactic acid bacteria beverage, fermented milk or the like from which various physiological effects are expected and for which the viable cell count is important. Moreover, to produce foods and drinks in which the flavor of a fermented material obtained by culturing a lactic acid bacterium is a problem, the bacterial strain to be used cannot be selected in view of the proliferative ability alone, and thus, in some cases, a lactic acid bacterium which produces a fermented material having an excellent flavor is selected and used even when the proliferative ability is poor.

Thus, for culturing a lactic acid bacterium, various growth-promoting substances are added to the medium in a general method, for the purpose of improving the culture efficiency. In general, examples of those which are considered effective as growth-promoting substances are Chlorella extract, iron salts, vitamins, protein degradation products containing amino acids and peptides, yeast extract and the like. The present applicant has also reported techniques of adding an extract of *Rubus suavissimus* S. Lee or a treated material thereof for improving the proliferative ability or the viability in culturing a lactic acid bacterium (PTLs 1 to 3).

Here, soybean polysaccharides are used as a thickener (stabilizer) in an acidic milk beverage using a culture of a lactic acid bacterium such as those described above. However, allergen-free products have recently spread also in various foods and drinks, and acidic milk beverages are no exception. Thus, it is desired that soybean-derived materials are not used also in acidic milk beverages to obtain soybean allergen-free products.

### Citation List

### Patent Literature

PTL 1: JP4739335B
PTL 2: JP5923360B
PTL 3: JP6633833B

### Summary of Invention

### Technical Problem

Accordingly, an object of the invention is to provide an acidic milk beverage containing live lactic acid bacterium that is an acidic milk beverage containing live lactic acid bacterium which is soybean allergen-free and which has improved viability of a lactic acid bacterium.

### Solution to Problem

As a result of extensive studies to solve the problem, the present inventors have found that, when a specific treated material of a pea polysaccharide is used in an acidic milk beverage containing live lactic acid bacterium, the acidic milk beverage becomes soybean allergen-free, and also the viability of a lactic acid bacterium is improved. The invention has been thus completed.

That is, the invention is an acidic milk beverage containing live lactic acid bacterium characterized by containing an ultrafiltration membrane-treated material and/or an ethanol-precipitated material of a pea polysaccharide.

Moreover, the invention is an agent for improving viability of a live lactic acid bacterium in an acidic milk beverage containing live lactic acid bacterium which is characterized by containing an ultrafiltration membrane-treated material and/or an ethanol-precipitated material of a pea polysaccharide as an active ingredient.

Furthermore, the invention is a method for improving viability of a live lactic acid bacterium in an acidic milk beverage containing live lactic acid bacterium which is characterized by adding an ultrafiltration membrane-treated material and/or an ethanol-precipitated material of a pea polysaccharide to an acidic milk beverage containing live lactic acid bacterium.

### Advantageous Effects of Invention

The acidic milk beverage containing live lactic acid bacterium of the invention is not only soybean allergen-free but also has improved viability of a lactic acid bacterium and thus can be drunk daily without worry, and various effects of the lactic acid bacterium can be thus obtained.

### Description of Embodiments

The acidic milk beverage containing live lactic acid bacterium of the invention (simply referred to as "the beverage of the invention" below) contains an ultrafiltration membrane-treated material and/or an ethanol-precipitated material of a pea polysaccharide. Here, the beverage of the invention is an acidic milk beverage containing a lactic acid bacterium in the form of live bacterium (an acidic milk beverage of the so-called live bacterium type) but is not an acidic milk beverage containing the lactic acid bacterium in the form of dead bacterium only through treatment such as pasteurization (an acidic milk beverage of the so-called dead bacterium type).

The pea polysaccharide used above is preferably extracted using a fiber fraction obtained by removing the protein fraction and the starch fraction contained in peas as a raw material. As the pH during the extraction, pH 3 to pH 12 is appropriate, and pH 4 to pH 10 is preferable, because hydrolysis of the polysaccharide is promoted under acidic conditions of less than pH 3 and because desorption and degradation of the polysaccharide is promoted at the alkaline side of more than pH 12. An acid or an alkali is added to adjust the pH in the range of pH 3 to pH 12 after adding water to the raw material, and then the pea polysaccharide is extracted at a temperature of preferably 60°C or higher and 150°C or lower, further preferably 80°C or higher and 130°C or lower. At a temperature lower than 60°C, the extraction efficiency of the pea polysaccharide is poor, and the extraction is less practical. At a temperature exceeding 150°C, the pea polysaccharide is sometimes hydrolyzed in the extraction process. The extraction period is generally 0.5 to three hours but can be freely adjusted depending on the state of the raw material, the temperature or the like. The acid or the alkali used is not particularly restricted. An acid such as hydrochloric acid, sulfuric acid, phosphoric acid, citric acid, tartaric acid, acetic acid and formic acid or an alkali such as sodium hydroxide, calcium hydroxide, sodium hydrogen carbonate, sodium carbonate and ammonia can be used. Moreover, one kind of cellulases, hemicellulases and pectinases having high purity which do not hydrolyze the pea polysaccharide or a combination thereof may be used for the extraction.

The extracted pea polysaccharide is subjected to the esterolysis described below after separating the insoluble fiber content. Although the pea polysaccharide can be dried directly, purification such as removal of protein, desalination and removal of a pigment component is desirably conducted to exhibit the function more. As the method for removing protein, the protein can be aggregated by pH adjustment and removed by physical separation means such as separation by pressure filtration, centrifugation and membrane separation. Moreover, the protein can be degraded using any protease and removed by adsorption using the degraded material using a dialysis membrane, active carbon or an ion-exchange or hydrophobic resin. As the desalination method, any method can be used as long as the method is a removal method thereof such as electrodialysis and an ion-exchange resin. As the method for removing a pigment component, any method such as methods for degrading a pigment component including ozone treatment and UV application can be used. One method thereof or a combination of two or more methods thereof is preferably used. The pea polysaccharide after the purification is subjected to any pasteurization, and a dried material is obtained by a method such as lyophilization, spray-drying and hot-air drying of an ethanol precipitate. When the ultrafiltration membrane treatment or the ethanol precipitation described below is conducted, a dried material may also be obtained by the above method after conducting the treatment subsequent to the purification.

When starch is contained in the raw material of the pea polysaccharide, the pea polysaccharide can be obtained as it is, but precipitates may be formed due to the starch when the pea polysaccharide is used for an acidic milk beverage containing live lactic acid bacterium. Accordingly, the starch is preferably removed in the stage of the raw material, the stage of the fiber separated from the raw material, the stage of the extraction of the pea polysaccharide or the stage after the extraction. The starch can be removed by one of or a combination of two or more of methods of degradation by an amylase, cooling precipitation and aggregation precipitation with an emulsifier. In the stage of the raw material, although dry fractionation can be used, wet fractionation is preferable, and the starch can be separated as starch particles by adding water to the crushed raw material, warming to a temperature at which the starch is not gelatinized and by centrifugal filtration. Moreover, the starch can also be degraded and removed by heating the water-added raw material to a temperature at which the starch is gelatinized or higher and treating with an amylase. In the stage of the fiber separated from the raw material of the pea polysaccharide, the starch can be degraded and removed by dispersing the fiber in water, warming to a temperature at which the starch is gelatinized and treating with an amylase. Examples of the method for removing the starch in the process of the extraction of the pea polysaccharide or after the extraction include a method by adding an amylase to the water-added raw material before the extraction, a method by adding an amylase to the slurry before the extraction and solid-liquid separation and a method by adding an amylase to the filtrate after solid-liquid separation.

Amylase is a generic term for enzymes which hydrolyze starch, and examples thereof include β-amylase, α-amylase, glucoamylase and pullulanase. These amylases with high purity may be used for the purpose, but a commercial amylase preparation containing one kind thereof or two or more kinds thereof mixed therein may also be used. Here, the starch can also be degraded and removed before or after the extraction of the raw material, the fiber or the pea polysaccharide by a chemical method such as acid hydrolysis, but because the pea polysaccharide is degraded at the same time, starch removal by enzymatic treatment is preferable.

Through a step of adjusting the degree of methylesterification to preferably 45% or less, more preferably 30% or less, the function of the pea polysaccharide above of dispersing protein becomes further excellent. The method for removing the methyl ester may be any method as long as the method can suppress sugar chain degradation of the pea polysaccharide and can degrade the ester. When the pea polysaccharide after the extraction is treated, esterolysis is possible by adding any alkali to an aqueous 1 mass% to 5 mass% pea polysaccharide solution to adjust to preferably pH 8 or more, more preferably pH 12 or more. The heating conditions are preferably 20°C or higher, more preferably 40°C or higher, and the heating period is preferably 10 minutes or longer, more preferably 30 minutes or longer, and is preferably four hours or shorter. When the pH during the extraction is at an alkaline side, the extraction and the esterolysis can also be conducted simultaneously. Moreover, the esterolysis may be conducted using a commercial pectin methylesterase or a commercial enzyme preparation containing the enzyme. Here, the esterolysis can be conducted at any stage in the preparation of the pea polysaccharide. An example thereof is ammonia treatment or the like of the raw material before the extraction, the slurry after the extraction, the pea polysaccharide solution after the solid-liquid separation, the purified solution or the powder after drying. As the alkali, any of sodium hydroxide, calcium hydroxide, sodium hydrogen carbonate, sodium carbonate, ammonia and the like can be used. Moreover, the degree of methylesterification is calculated by measuring the galacturonic acid amount and the galacturonic acid methyl ester amount by the Doesburg titration. Degree of methylesterification = galacturonic acid methyl ester/total galacturonic acid × 100(%)

The degree of methylesterification of the constituent galacturonic acid of the pea polysaccharide above is preferably 45% or less, more preferably 30% or less.

The pea polysaccharide above is subjected to ultrafiltration membrane treatment to obtain an ultrafiltration membrane-treated material. The ultrafiltration membrane treatment is not particularly limited but may be conducted, for example, using an ultrafiltration membrane having a molecular cutoff of 1 to 150 kDa, preferably 3 to 100 kDa.

Moreover, the pea polysaccharide above is subjected to ethanol precipitation to obtain an ethanol-precipitated material. The ethanol precipitation is not particularly limited but may be conducted, for example, by adding an aqueous ethanol solution of 50 to 100 mass%, preferably 60 to 100 mass%, to the pea polysaccharide to 40 to 95 mass%, preferably 50 to 95 mass%, precipitating at 0 to 100°C, preferably 10 to 80°C, for one minute to three hours and then conducting filtration, centrifugation or the like.

A preferable ultrafiltration membrane-treated or ethanol precipitated material of a pea polysaccharide used in the beverage of the invention is a material which is obtained from the pea seeds described in JP6131853B or from a fiber fraction obtained by removing the protein fraction and the starch fraction contained in the pea seeds as a raw material by degrading in an aqueous system of pH 3 to pH 12 in such a manner that arabinose, galactose, glucose, rhamnose, xylose, fucose and galacturonic acid are contained as the constituent saccharides and that the degree of methylesterification becomes preferably 45% or less. Such a material corresponds to pea pectins A, G, H, J and K described in the patent publication.

For the beverage of the invention, of the ultrafiltration membrane-treated material and the ethanol-precipitated material, the ultrafiltration membrane-treated material is preferably used in view of the safety and the like.

The amount of the ultrafiltration membrane-treated material and/or the ethanol-precipitated material of the pea polysaccharide contained in the beverage of the invention is not particularly limited but is, for example, 0.05 to 2.0 mass%, preferably 0.1 to 1.0 mass%, more preferably 0.25 to 0.50 mass%.

The beverage of the invention is an acidic milk beverage containing a live lactic acid bacterium obtained by culturing the lactic acid bacterium in a medium. The viable cell count of the lactic acid bacterium is not particularly limited but is, for example, 1.0×10⁷ cfu/ml or more, preferably 1.0×10⁸ cfu/ml or more. The beverage of the invention is suitable for the so-called high cell count type particularly having a viable cell count of the lactic acid bacterium of 1.0×10⁹ cfu/ml or more.

The medium for culturing the lactic acid bacterium is not particularly limited, but examples thereof include a mammalian milk medium composed of raw milk such as cow's milk, goat's milk, mare milk and sheep's milk, a dairy product such as skim milk powder, whole milk powder and fresh cream or the like, plant-derived liquid milk and a various synthetic media. The medium may contain a component which is used for a general medium for culturing a lactic acid bacterium. Examples of such a component include vitamins such as vitamin A, B vitamins, vitamin C and vitamin E, various peptides, amino acids and salts of calcium, magnesium and the like.

Moreover, a known culture adjuvant such as the extract of *Rubus suavissimus* S. Lee or the treated material thereof described in PTLs 1 to 3 may be contained in the medium.

The lactic acid bacterium is not particularly limited as long as the lactic acid bacterium can be cultured in the medium, but examples thereof include bacteria of the genus *Lacticaseibacillus* such as *Lacticaseibacillus paracasei* and *Lacticaseibacillus casei,* bacteria of the genus *Lactobacillus* such as *Lactobacillus gasseri, Lactobacillus acidophilus, Lactobacillus helveticus, Lactobacillus delbrueckii* subsp. *bulgaricus, Lactobacillus delbrueckii* subsp. *delbrueckii* and *Lactobacillus johnsonii,* bacteria of the genus *Ligilactobacillus* such as *Ligilactobacillus salivarius,* bacteria of the genus *Limosilactobacillus* such as *Limosilactobacillus fermentum,* bacteria of the genus *Liquorilactobacillus* such as *Liquorilactobacillus mali,* bacteria of the genus *Lactiplantibacillus* such as *Lactiplantibacillus plantarum,* bacteria of the genus *Streptococcus* such as *Streptococcus thermophilus,* bacteria of the genus *Lactococcus* such as *Lactococcus lactis* subsp. *lactis, Lactococcus lactis* subsp. *cremoris, Lactococcus plantarum, Lactococcus raffinolactis* and *Lactococcus cremoris* and bacteria of the genus *Enterococcus* such as *Enterococcus faecalis* and *Enterococcus faecium.* One kind or two or more kinds of the lactic acid bacteria can be used. Of these, the bacteria of the genus *Lacticaseibacillus* are preferable, and *Lacticaseibacillus paracasei* is more preferable. *Lacticaseibacillus paracasei* YIT 9029 (FERM BP-1366) is particularly preferable.

In the invention, the lactic acid bacteria used for the culture also include bacteria belonging to the genus *Bifidobacterium,* which are anaerobic bacteria, in addition to those which are generally called lactic acid bacteria such as the above bacteria. Such bacteria belonging to the genus *Bifidobacterium* are not particularly limited and may be *Bifidobacterium breve, Bifidobacterium pseudocatenulatum, Bifidobacterium bifidum, Bifidobacterium longum, Bifidobacterium infantis, Bifidobacterium adolescentis, Bifidobacterium catenulatum, Bifidobacterium angulatum, Bifidobacterium gallicum, Bifidobacterium lactis, Bifidobacterium animalis* and the like. Of these, *Bifidobacterium bifidum, Bifidobacterium breve* and the like are preferable, and in particular, *Bifidobacterium bifidum* is more preferable. An example of *Bifidobacterium bifidum* is, for example, Bifidobacterium bifidum YIT10347 (FERM BP-10613, deposition date: June 23, 2005) or the like.

In this regard, *Lacticaseibacillus paracasei* YIT 9029 was previously classified as *Lactobacillus casei* but reclassified as *Lacticaseibacillus paracasei* after the recent reclassification of the genus *Lactobacillus* (Zheng et al., A taxonomic note on the genus Lactobacillus: Description of 23 novel genera, emended description of the genus Lactobacillus Beijerinck 1901, and union of Lactobacillaceae and Leuconostocaceae. Int. J. Syst. Evol. Microbiol. 2020 Apr; 70(4):2782-2858 DOI 10.1099/ijsem.0.004107). *Lactobacillus casei* YIT 9029 was deposited for an international deposit as Lactobacillus casei YIT 9029 (FERM BP-1366, accession date: May 1, 1981) to an international depositary authority under the Budapest Treaty, International Patent Organism Depositary, National Institute of Technology and Evaluation, International Patent Organism Depositary (#120, 2-5-8 Kazusakamatari, Kisarazu-shi, Chiba, Japan 292-0818).

The conditions for culturing the lactic acid bacterium in the medium are not particularly limited, but examples thereof include conditions of inoculating the lactic acid bacterium in the medium in such a manner that the bacterial count in the medium becomes around 1.0 × 10³ to 1.0 × 10⁹ cfu/ml and culturing at a temperature around 30 to 40°C for around one to seven days. Moreover, regarding the culturing conditions here, a method which is suitable for culturing the lactic acid bacterium used may be appropriately selected from static method, stirring, shaking, aeration and the like.

The pH of the beverage of the invention is not particularly limited, but in view of the flavor as the acidic milk beverage and the stability, the pH is more preferably adjusted to 4.2 or less and particularly preferably adjusted to 3.0 to 4.0. For the pH adjustment, an organic acid which is generally used for a food may be used. Examples of such an organic acid include malic acid, succinic acid, citric acid, tartaric acid and the like. One kind or two or more kinds of the organic acids can be used, but one kind or two or more kinds selected from malic acid, citric acid and tartaric acid are preferably used.

The solids-not-fat content of the beverage of the invention is not particularly limited, but in view of the flavor as the acidic milk beverage and the stability, the solids-not-fat content is preferably 4.0 mass% or less or 3.0 to 4.0 mass%. The solids-not-fat content can be adjusted by adjusting the amount of the skim milk powder used.

The beverage of the invention may further contain a saccharide such as sucrose, glucose, fructose and sweeteners having high sweetness, an aroma, an emulsifier, a vitamin or the like.

Moreover, the beverage of the invention may further contain a thickener (stabilizer) such as pectin, succinoglycan, sodium carboxymethyl cellulose and gellan gum as long as the effects of the invention are not impaired. Here, of course, thickeners (stabilizers) obtained from soybeans as a raw material are not contained.

The beverage of the invention can be produced by adding the ultrafiltration membrane-treated material and/or the ethanol-precipitated material of the pea polysaccharide to an acidic milk beverage containing the live lactic acid bacterium obtained by culturing the lactic acid bacterium in the medium. The timing for adding the ultrafiltration membrane-treated material and/or the ethanol-precipitated material of the pea polysaccharide is not particularly limited and may be the same as the timing for adding a known thickener (stabilizer), but for example, addition with a saccharide, an aroma, an emulsifier, a vitamin or the like after culturing the lactic acid bacterium in the medium is preferable.

The beverage of the invention thus obtained is a soybean allergen-free acidic milk beverage, and the viability of the lactic acid bacterium is improved. Here, that the viability of the lactic acid bacterium is improved means that the survival rate after long-term (28 days or longer) storage is 90% or more of the survival rate of a case in which a soybean polysaccharide that achieves equivalent stability is used as a stabilizer. In some cases, when the bacterial count of a lactic acid bacterium is measured, the count is increased after storage compared to that before storage due to the measurement error or the like or depending on the initial bacterial count, but even in such a case, whether the viability is improved is determined in the invention by comparing with the survival rate of a case in which a soybean polysaccharide is used as a stabilizer to obtain equivalent stability as the above. In this manner, the ultrafiltration membrane-treated material and/or the ethanol-precipitated material of the pea polysaccharide can improve the viability of the lactic acid bacterium in an acidic milk beverage containing live lactic acid bacterium.

### Examples

The invention is explained in detail below referring to Examples of the invention, but the invention is not limited to the Examples.

### Production Example 1

### Preparation of Pea Polysaccharides (Untreated Material):

After hulling 50 kg of pea seeds (yellow peas), five times the amount of water was added, and the peas were immersed for 24 hours. The seeds were crushed with a homomixer (5,000 rpm, 30 minutes), and protein and starch were extracted. Components which were dispersed in water, such as protein and starch, were removed using a centrifugal filter at 1,500×g for 20 minutes, and the fiber was collected. Further, five times the amount of water was added to the fiber, followed by stirring with a homomixer (3,000 rpm, 30 minutes), and the fiber was collected by centrifugal filtration (1,500×g, 20 minutes). The operation was repeated twice, and 10 kg of pea fiber was obtained by lyophilization. Eighty parts of the pea fiber were dispersed in 920 parts of water, and after adjusting the pH to 5 using hydrochloric acid, the dispersion was heated at 120°C for 90 minutes to extract the pea polysaccharides. Insoluble fiber was removed by centrifugation (5,000 rpm, 30 minutes), and the supernatant was collected. The supernatant was warmed to 40°C, adjusted to pH 12 with an alkali and maintained for 60 minutes for demethylesterification. After adjusting to pH 7 by adding hydrochloric acid and warming to 60°C, an amylase (BAN480L Novozymes) which corresponded to 0.1 weight% of the solid content was added, and the starch was degraded for an hour. Hydrochloric acid was added to the liquid after the starch degradation to adjust to pH 5, and insoluble components were removed by centrifugation (5,000 rpm, 30 minutes). The supernatant was collected, and after desalination, pea polysaccharides (untreated material) (2.4 mass% water content, 3.5 mass% crude protein, 88.4 mass% carbohydrate, 5.7 mass% ash content, degree of methylesterification of 15.0%) were obtained by lyophilization.

The absolute molecular weight of the polymeric components having a molecular weight of 10,000 or more of the pea polysaccharides was measured by static light scattering (HPLC-MALLS). The analysis conditions of the gel filtration chromatography of the HPLC are as follows.

An aqueous 1 weight% solution of the pea polysaccharides (an aqueous 50 mM sodium acetate solution, pH 5.0) was prepared and filtered with a 0.8 µm filter, and the filtrate was subjected to analysis. The filtrate in a volume of 20 µL was added to a TSK-gel G-6000PWXL column (Tosoh Corporation; φ7.8 mm × 300 mm) which was equilibrated in advance with an aqueous 50 mM sodium acetate solution at pH 5.0 at 40°C and separated at a flow rate of 1.0 mL/min. The separated polysaccharides were detected with a differential refractometer (RI detector), and the molecular weight was calculated with standard pullulan P-82 (Showa Denko K.K.). As a result, the average absolute molecular weight was 850,000.

### Production Example 2

### Preparation of Pea Polysaccharides (Untreated Material):

Pea polysaccharides (untreated material) (1.8 mass% water content, 6.2 mass% crude protein, 88.5 mass% carbohydrate, 3.5 mass% ash content, degree of methylesterification of 14.8%) were obtained by the same method as that of Production Example 1.

### Production Example 3

### Preparation of Ultrafiltration Membrane-Treated Material (UF Membrane-Treated Material) of Pea Polysaccharides:

By degrading starch with an amylase in the same manner as in Production Example 1, then adjusting to pH 5 with hydrochloric acid, removing insoluble components by centrifugation (5,000 rpm, 30 minutes), collecting the supernatant, treating with an ultrafiltration membrane having a molecular cutoff of 30 kDa and lyophilizing, an ultrafiltration membrane-treated material (UF membrane-treated material) of pea polysaccharides (3.2 mass% water content, 3.9 mass% crude protein, 87.3 mass% carbohydrate, 5.6 mass% ash content, degree of methylesterification of 15.0%) was obtained.

### Production Example 4

### Preparation of Ultrafiltration Membrane-Treated Material (UF Membrane-Treated Material) of Pea Polysaccharides:

An ultrafiltration membrane-treated material (UF membrane-treated material) of pea polysaccharides (4.8 mass% water content, 5.1 mass% crude protein, 81.1 mass% carbohydrate, 9.0 mass% ash content, degree of methylesterification of 14.8%) was obtained by the same method as that of Production Example 3.

### Production Example 5

### Preparation of Ethanol-Precipitated Material of Pea Polysaccharides:

By degrading starch with an amylase in the same manner as in Production Example 1, then adjusting to pH 5 with hydrochloric acid, adding 90 weight% ethanol to the supernatant obtained by removing insoluble components by centrifugation in such a manner that the ethanol concentration became 60 weight% to precipitate the pea polysaccharides, washing with 90 weight% water-containing ethanol and air-drying the obtained precipitate, an ethanol-precipitated material of pea polysaccharides (8.1 mass% water content, 6.6 mass% crude protein, 80.7 mass% carbohydrate, 4.6 mass% ash content, degree of methylesterification of 14.8%) was obtained.

### Example 1

### Preparation of Acidic Milk Beverages Containing Live Lactic Acid Bacterium (High Cell Count Type):

A starter of *Lacticaseibacillus paracasei* (strain YIT 9029) was inoculated at 0.5% (viable cell count of 5.0×10⁶ cfu/ml) in a 15 mass% skim milk powder medium (containing 1.8 mass% glucose and 3.0 mass% fructose) and cultured at 37°C until the pH became 3.6, and an acidic raw material milk was thus obtained. Next, syrups (containing 12 mass% sugar) which contained the pea polysaccharides (untreated material) prepared in Production Example 1 or the ultrafiltration membrane-treated material (UF membrane-treated material) of the pea polysaccharides prepared in Production Example 3 were prepared separately. Mixtures obtained by mixing 24 parts by weight of the acidic raw material milk and 76 parts by weight of the syrups were homogenized at 15 MPa, and thus acidic milk beverages containing live lactic acid bacterium were obtained. The solids-not-fat contents of the acidic milk beverages were 3.7 mass%, and the pH and the polysaccharide contents soon after the production (0 day of storage) are as shown in Table 1. Moreover, as comparison, a similar acidic milk beverage containing live lactic acid bacterium was obtained using conventional soybean polysaccharides (manufactured by San-Ei Gen F.F.I., Inc.).

The *Lacticaseibacillus paracasei* (strain YIT 9029) used above is *Lactobacillus casei* (strain YIT 9029) according to the previous classification.

After the acidic milk beverages containing live lactic acid bacterium were stored at 10°C for 30 days, the amounts of whey off (the height (mm) of the transparent layer generated in the upper part of the beverage) and the precipitate amounts (the weight of the precipitates at the container bottom/the weight of the whole content × 100(%)) were measured. The amounts of whey off in the containers were directly measured. Moreover, the precipitate amounts were determined visually by the following criteria. Further, the viable cell counts, the pH and the titratable acidities were measured by known methods. Furthermore, the survival rates were calculated as follows. The results are shown in Table 1.

### <Assessment Criteria of Precipitate Amounts>

### (Assessment) (Contents)

+: Precipitates were formed at the entire bottom of the container.
±: Precipitates were formed around the circumference of the bottom of the container.
-: No precipitates were formed at the bottom of the container.

### <Survival Rates>

The survival rates were determined by the following equation from the measured viable cell counts. Survival Rate = (viable cell count on last day of storage at 10°C/viable cell count soon after production (day 0 of storage) × 100(%)

**[Table 1]**

| Stabilizer | | Soybean polysaccharides | Pea polysaccharides | |
|---|---|---|---|---|
| | | | Untreated material (Production Example 1) | UF membrane-treated material (Production Example 3) |
| Water content | | -- | 2.4% | 3.2% |
| Crude protein | | -- | 3.5% | 3.9% |
| Carbohydrate (containing fiber) | | -- | 88.4% | 87.3% |
| Ash content | | -- | 5.7% | 5.6% |
| Added amount in beverage (w/v) | | 0.30% | 0.375% | |
| Whey off (mm) | Day 14 | 6.0 | 6.0 | 6.0 |
| | Day 21 | 8.0 | 9.0 | 8.0 |
| | Day 30 | 9.0 | 11.0 | 9.0 |
| Precipitate amount | Day 14 | 0.80% | 1.00% | 0.90% |
| | Day 21 | 0.90% | 1.20% | 0.80% |
| | Day 30 | 1.00% | 1.30% | 1.10% |
| Precipitate amount (visual observation) | Day 14 | ± | ± | ± |
| | Day 21 | ± | ± | ± |
| | Day 30 | ± | ± | ± |
| Viable cell count (cfu/ml) | Day 0 | 1.9E+09 | 1.8E+09 | 1.8E+09 |
| | Day 14 | 1.9E+09 | 1.9E+09 | 1.9E+09 |
| | Day 21 | 1.9E+09 | 1.7E+09 | 1.9E+09 |
| | Day 30 | 1.6E+09 | 1.3E+09 | 1.5E+09 |
| Survival rate* | Day 30/Day 0 | 82% | 72% | 83% |
| | | (100%) | (88%) | (101%) |
| pH | Day 0 | 3.71 | 3.69 | 3.71 |
| | Day 14 | 3.62 | 3.62 | 3.63 |
| | Day 21 | 3.54 | 3.53 | 3.54 |
| | Day 30 | 3.50 | 3.51 | 3.52 |
| Titratable acidity (ml/9g) | Day 0 | 6.10 | 6.20 | 6.10 |
| | Day 14 | 7.10 | 7.10 | 7.00 |
| | Day 21 | 7.60 | 7.50 | 7.40 |
| | Day 30 | 7.97 | 7.81 | 7.78 |

| | | | | |
|---|---|---|---|---|
| * The values in the parenthesis are the percentages of the survival rates, where the survival rate with the soybean polysaccharides is regarded as 100%. Except for the survival rates, % is mass%. | | | | |

From the results, when the UF membrane-treated material was used, physical stability and viability of the live bacterium which were equivalent to those of the case using the soybean polysaccharides were observed. Moreover, when the UF membrane-treated material was used, the viability of the live bacterium could be improved significantly compared to the case using the untreated material.

### Example 2

### Preparation of Acidic Milk Beverages Containing Live Lactic Acid Bacterium (High Cell Count Type):

A starter of *Lacticaseibacillus paracasei* (strain YIT 9029) was inoculated at 0.5 mass% (viable cell count of 5.0×10⁶ cfu/ml) in a 15 mass% skim milk powder medium (containing 1.8 mass% glucose and 3.0 mass% fructose) and cultured at 37°C until the pH became 3.6, and an acidic raw material milk was thus obtained. Next, syrups (containing 12 mass% sugar) which contained the pea polysaccharides (untreated material) prepared in Production Example 2 or the ethanol-precipitated material of the pea polysaccharides prepared in Production Example 5 were prepared separately. Mixtures obtained by mixing 24 parts by weight of the acidic raw material milk and 76 parts by weight of the syrups were homogenized at 15 MPa, and thus acidic milk beverages containing live lactic acid bacterium were obtained. The solids-not-fat contents of the acidic milk beverages were 3.7 mass%, and the pH and the polysaccharide contents soon after the production (0 day of storage) are as shown in Table 2. Moreover, as comparison, a similar acidic milk beverage containing live lactic acid bacterium was obtained using conventional soybean polysaccharides (manufactured by San-Ei Gen F.F.I., Inc.).

After the acidic milk beverages containing live lactic acid bacterium were stored at 10°C for 30 days, the amounts of whey off (the height (mm) of the transparent layer generated in the upper part of the beverage) and the precipitate amounts (the weight of the precipitates at the container bottom/the weight of the whole content × 100(%)) were measured. The amounts of whey off in the containers were directly measured. Moreover, the precipitate amounts were determined visually by the same criteria as those in Example 1. Further, the viable cell counts, the pH and the titratable acidities were measured by known methods. Furthermore, the survival rates were calculated in the same manner as in Example 1. The results are shown in Table 2.

**[Table 2]**

| Stabilizer | | Soybean polysaccharides | Pea polysaccharides | |
|---|---|---|---|---|
| | | | Untreated material (Production Example 2) | Ethanol-precipitated material (Production Example 5) |
| Water content | | -- | 1.8% | 8.1% |
| Crude protein | | -- | 6.2% | 6.6% |
| Carbohydrate (containing fiber) | | -- | 88.5% | 80.7% |
| Ash content | | -- | 3.5% | 4.6% |
| Added amount in beverage (w/v) | | 0.30% | 0.375% | |
| Whey off (mm) | Day 10 | 6.0 | 6.0 | 6.0 |
| | Day 17 | 10.0 | 11.0 | 11.0 |
| | Day 21 | 16.0 | 16.0 | 15.0 |
| | Day 25 | 15.0 | 16.0 | 15.0 |
| | Day 30 | 15.0 | 15.0 | 15.0 |
| Precipitate amount | Day 10 | 0.73% | 0.59% | 0.69% |
| | Day 17 | 0.93% | 0.77% | 0.75% |
| | Day 21 | 0.99% | 0.97% | 1.04% |
| | Day 25 | 1.11% | 1.15% | 1.02% |
| | Day 30 | 0.95% | 0.97% | 0.89% |
| Precipitate amount (visual observation) | Day 10 | - | - | - |
| | Day 17 | ± | ± | ± |
| | Day 21 | ± | ± | ± |
| | Day 25 | ± | ± | ± |
| | Day 30 | ± | ± | ± |
| Viable cell count (cfu/ml) | Day 0 | 1.6E+09 | 1.7E+09 | 1.7E+09 |
| | Day 10 | 1.6E+09 | 1.6E+09 | 1.7E+09 |
| | Day 17 | 1.6E+09 | 1.5E+09 | 1.7E+09 |
| | Day 21 | 1.6E+09 | 1.4E+09 | 1.4E+09 |
| | Day 25 | 1.4E+09 | 1.1E+09 | 1.3E+09 |
| | Day 30 | 1.2E+09 | 1.1E+09 | 1.2E+09 |
| Survival rate* | Day 30/Day 0 | 75% | 65% | 71% |
| | | (100%) | (86%) | (94%) |
| pH | Day 0 | 3.69 | 3.67 | 3.68 |
| | Day 10 | 3.62 | 3.61 | 3.61 |
| | Day 17 | 3.59 | 3.58 | 3.58 |
| | Day 21 | 3.57 | 3.57 | 3.58 |
| | Day 25 | 3.56 | 3.56 | 3.56 |
| | Day 30 | 3.54 | 3.54 | 3.55 |
| Titratable acidity (ml/9g) | Day 0 | 6.18 | 6.16 | 6.11 |
| | Day 10 | 6.83 | 6.82 | 6.78 |
| | Day 17 | 7.25 | 7.22 | 7.14 |
| | Day 21 | 7.36 | 7.29 | 7.19 |
| | Day 25 | 7.51 | 7.39 | 7.31 |
| | Day 30 | 7.60 | 7.47 | 7.41 |

| | | | | |
|---|---|---|---|---|
| * The values in the parenthesis are the percentages of the survival rates, where the survival rate with the soybean polysaccharides is regarded as 100%. Except for the survival rates, % is mass%. | | | | |

From the results, when the ethanol-precipitated material was used, physical stability and viability of the live bacterium which were equivalent to those of the case using the soybean polysaccharides were observed. Moreover, when the ethanol-precipitated material was used, the viability of the live bacterium could be improved significantly compared to the case using the untreated material.

### Example 3

### Preparation of Acidic Milk Beverages Containing Live Lactic Acid Bacterium:

A 15 mass% skim milk powder medium (containing 3.5 mass% glucose) was pasteurized at 132°C for three seconds, and a starter of *Lacticaseibacillus paracasei* (strain YIT 9029) was inoculated at 0.5 mass% (viable cell count of 5.0×10⁶ cfu/ml) and cultured at 37°C until the pH became 3.6. Thus, an acidic raw material milk was obtained. Next, syrups (containing 5 mass% high-fructose corn syrup) which contained the ultrafiltration membrane-treated material (UF membrane-treated material) of the pea polysaccharides prepared in Production Example 4 were prepared separately. Mixtures obtained by mixing 24 parts by weight of the acidic raw material milk and 76 parts by weight of the syrups were homogenized at 15 MPa, and thus acidic milk beverages containing live lactic acid bacterium were obtained. The solids-not-fat contents of the acidic milk beverages were 3.3 mass%, and the pH and the polysaccharide contents soon after the production (0 day of storage) are as shown in Table 3. Moreover, as comparison, a similar acidic milk beverage containing live lactic acid bacterium was obtained using conventional soybean polysaccharides (manufactured by San-Ei Gen F.F.I., Inc.).

After the acidic milk beverages containing live lactic acid bacterium were stored at 10°C for 28 days, the amounts of whey off (the height (mm) of the transparent layer generated in the upper part of the beverage) and the precipitate amounts (the weight of the precipitates at the container bottom/the weight of the whole content × 100(%)) were measured. The amounts of whey off in the containers were directly measured. Moreover, the precipitate amounts were determined visually by the same criteria as those in Example 1. Further, the viable cell counts, the pH and the titratable acidities were measured by known methods. Furthermore, the survival rates were calculated in the same manner as in Example 1. The results are shown in Table 3.

**[Table 3]**

| Stabilizer | | Soybean polysaccharides | Pea polysaccharides | |
|---|---|---|---|---|
| | | | UF membrane-treated material (Production Example 4) | |
| Water content | | -- | 4.8% | |
| Crude protein | | -- | 5.1% | |
| Carbohydrate (containing fiber) | | -- | 81.1% | |
| Ash content | | -- | 9.0% | |
| Added amount in beverage (w/v) | | 0.30% | 0.40% | 0.20% |
| Whey off (mm) | Day 7 | 5.0 | 5.0 | 7.0 |
| | Day 14 | 10.0 | 10.0 | 14.0 |
| | Day 21 | 15.0 | 15.0 | 20.0 |
| | Day 28 | 16.0 | 16.0 | 32.0 |
| Precipitate amount | Day 7 | 0.69% | 0.63% | 0.70% |
| | Day 14 | 0.57% | 0.56% | 0.57% |
| | Day 21 | 1.49% | 1.22% | 1.31% |
| | Day 28 | 1.59% | 1.26% | 1.55% |
| Precipitate amount (visual observation) | Day 7 | ± | ± | ± |
| | Day 14 | + | + | + |
| | Day 21 | + | + | + |
| | Day 28 | + | + | + |
| Viable cell count (cfu/ml) | Day 0 | 7.0E+08 | 7.5E+08 | 7.6E+08 |
| | Day 7 | - | - | - |
| | Day 14 | - | - | - |
| | Day 21 | - | - | - |
| | Day 28 | 8.1E+08 | 8.2E+08 | 7.9E+08 |
| Survival rate* | Day 28/Day 0 | 116% | 109% | 104% |
| | | (100%) | (94%) | (90%) |
| pH | Day 0 | 3.72 | 3.73 | 3.70 |
| | Day 7 | 3.65 | 3.65 | 3.63 |
| | Day 14 | 3.60 | 3.60 | 3.58 |
| | Day 21 | 3.54 | 3.54 | 3.52 |
| | Day 28 | 3.48 | 3.48 | 3.46 |
| Titratable acidity (ml/9g) | Day 0 | 5.41 | 5.33 | 5.34 |
| | Day 7 | 5.80 | 5.81 | 5.81 |
| | Day 14 | 6.35 | 6.39 | 6.37 |
| | Day 21 | 6.86 | 6.81 | 6.80 |
| | Day 28 | 7.22 | 7.24 | 7.32 |

| | | | | |
|---|---|---|---|---|
| * The values in the parenthesis are the percentages of the survival rates, where the survival rate with the soybean polysaccharides is regarded as 100%. Except for the survival rates, % is mass%. | | | | |

From the results, when the UF membrane-treated material was used at 0.40%, physical stability and viability of the live bacterium which were equivalent to those of the case using the soybean polysaccharides were observed. Moreover, when the UF membrane-treated material was used at 0.20%, although the whey off value increased slightly, there was no problem in view of the stability, and results of the viability of the live bacterium which were equivalent to those of the case using the soybean polysaccharides were observed.

### Industrial Applicability

The acidic milk beverage containing live lactic acid bacterium of the invention can be used for promoting the health.

| | | |
|---|---|---|
| 0-1 | Form PCT/RO/134 | |
| | The indications relating to deposited microorganism(s) or other biological material (PCT Rule 13*bis*) were | |
| 0-1-1 | prepared using | JPO-PAS |
| | | i480 |
| 0-2 | International Application No. | |
| 0-3 | Applicant's or agent's file reference | PF-230003-WO |
| | | |
| 1 | The indications made below relate to the microorganism(s) or the biological material described in the detailed description of the invention. | |
| 1-1 | Paragraph number | 0031 |
| 1-3 | Indication of deposit | |
| 1-3-1 | Name of depositary institution | IPOD International Patent Organism Depositary, National Institute of Technology and Evaluation, International Patent Organism Depositary (NITE-IPOD) |
| 1-3-2 | Address of depositary institution | #120, 2-5-8 Kazusakamatari, Kisarazu-shi, Chiba, 292-0818, Japan |
| 1-3-3 | Date of deposit | May 1, 1981 (01.05.1981) |
| 1-3-4 | Accession number | IPOD FERM BP-1366 |
| 1-5 | Designated states for which the indications are made | All designated states |
| 2 | The indications made below relate to the microorganism(s) or the biological material described in the detailed description of the invention. | |
| 2-1 | Paragraph number | 0030 |
| 2-3 | Indication of deposit | |
| 2-3-1 | Name of depositary institution | IPOD International Patent Organism Depositary, National Institute of Technology and Evaluation, International Patent Organism Depositary (NITE-IPOD) |
| 2-3-2 | Address of depositary institution | #120, 2-5-8 Kazusakamatari, Kisarazu-shi, Chiba, 292-0818, Japan |
| 2-3-3 | Date of deposit | June 23, 2005 (23.06.2005) |
| 2-3-4 | Accession number | IPOD FERM BP-10613 |
| 2-5 | Designated states for which the indications are made | All designated states |

| For Receiving Office Use | | |
|---|---|---|
| 0-4 | This sheet was received with the international application (Yes/No). | ✔ |
| 0-4-1 | Authorized officer | Yuka Hirakawa |

| For International Bureau Use | | |
|---|---|---|
| 0-5 | Date on which this sheet was received by the international bureau | |
| 0-5-1 | Authorized officer | |

Replacement Sheet (Rule 26)

## Claims

1. An acidic milk beverage containing live lactic acid bacterium **characterized by** containing an ultrafiltration membrane-treated material and/or an ethanol-precipitated material of a pea polysaccharide.

2. The acidic milk beverage containing live lactic acid bacterium according to claim 1 which has pH 4.2 or less.

3. The acidic milk beverage containing live lactic acid bacterium according to claim 1 or 2 which has a solids-not-fat content of 4.0 mass% or less.

4. An agent for improving viability of a live lactic acid bacterium in an acidic milk beverage containing live lactic acid bacterium which is **characterized by** containing an ultrafiltration membrane-treated material and/or an ethanol-precipitated material of a pea polysaccharide as an active ingredient.

5. A method for improving viability of a live lactic acid bacterium in an acidic milk beverage containing live lactic acid bacterium which is **characterized by** adding an ultrafiltration membrane-treated material and/or an ethanol-precipitated material of a pea polysaccharide to an acidic milk beverage containing live lactic acid bacterium.
